# EUROPEAN PATENT SPECIFICATION

(11) **EP 1 074 264 B1**
(45) Date of publication and mention of the grant of the patent: **17.09.2008**
(21) Application number: 99912129.6
(22) Date of filing: 06.04.1999
(51) Int. Cl.: A61K 38/18, C07K 14/475

(54) **NEOVASCULARIZATION INHIBITORS**
INHIBITOREN FÜR DIE GEFÄSSNEUBILDUNG
INHIBITEURS DE NEOVASCULARISATION

(30) Priority: 28.04.1998 JP 13468198
(43) Date of publication of application: 07.02.2001
(73) Proprietor: Nakamura, Toshikazu, Kyoto 606-8333 (JP)
(72) Inventor: Nakamura, Toshikazu, Kyoto 606-8333 (JP)
(74) Representative: HOFFMANN EITLE
(86) International application number: PCT/JP1999/001834
(87) International publication number: WO 1999/055361

(56) References cited:
- EP-A1- 0 461 560
- WO-A-95/29242
- WO-A-96/35774
- WO-A-96/40914
- DATE K. et al., "HGF/NK4 is a Specific Antagonist for Pleiotrophic Actions of Hepatocyte Growth Factor", FEBS LETT., 1997, Vol. 420, No. 1, pages 1-6, XP002920323.
- BUSSOLINO F. et al., "Hepatocyte Growth Factor is a Potent Angiogenic Factor Which Stimulates Endothelial Cell Motility and Growth", J. CELL. BIOL., 1992, Vol. 119, No. 3, pages 629-641, XP002920324.
- NISHIMURA M. et al., "Serum Hepatocyte Growth Factor as a Possible Indicator of Vascular Lesions", RINSHO BYORI, 1997, Vol. 45, No. 9, pages 831-836, Refer to Abstract, Study (see also CHEMICAL ABSTRACT, 1997, Vol. 127, No. 18, Abst. No. 246281y), XP002920325.

### SEQUENCE LISTING

<110> Nakamura, Toshikazu
<120> Inhibitor of Vascularization
<130> P99-10
<140>
   <141>
<150> JP P1998/134681
   <151> 1998-04-28
<160> 2
<170> PatentIn Ver. 2.0
<210> 1
   <211> 447
   <212> PRT
   <213> Human
<220>
   <221> MOD_RES
   <222> (1)
   <223> pyroglutamate
<220>
   <221> CHAIN
   <222> (1).. (447)
   <223> N-terminal region of alpha-chain in HGF (PyrGlu32-Val478/HGF)
<300>
   <301> Nakamura, Tshikazu
   <303> Nature
   <304> 342
   <306> 440-443
   <307> 1989
<400> 1
   Xaa Arg Lys Arg Arg Asn Thr Ile His Glu Phe Lys Lys Ser Ala Lys
   1               5                   10                  15
   Thr Thr Leu Ile Lys Ile Asp Pro Ala Leu Lys Ile Lys Thr Lys Lys
               20                  25                  30
   Val Asn Thr Ala Asp Gln Cys Ala Asn Arg Cys Thr Arg Asn Lys Gly
           35                  40                  45
   Leu Pro Phe Thr Cys Lys Ala Phe Val Phe Asp Lys Ala Arg Lys Gln
       50                  55                  60
   Cys Leu Trp Phe Pro Phe Asn Ser Met Ser Ser Gly Val Lys Lys Glu
   65                  70                  75                  80
   Phe Gly His Glu Phe Asp Leu Tyr Glu Asn Lys Asp Tyr Ile Arg Asn
                   85                  90                  95
   Cys Ile Ile Gly Lys Gly Arg Ser Tyr Lys Gly Thr Val Ser Ile Thr
   100 105 110
   Lys Ser Gly Ile Lys Cys Gln Pro Trp Ser Ser Met Ile Pro His Glu
   115 120 125
   His Ser Phe Leu Pro Ser Ser Tyr Arg Gly Lys Asp Leu Gln Glu Asn
   130 135 140
   Tyr Cys Arg Asn Pro Arg Gly Glu Glu Gly Gly Pro Trp Cys Phe Thr
   145 150 155 160
   Ser Asn Pro Glu Val Arg Tyr Glu Val Cys Asp Ile Pro Gln Cys Ser
   165 170 175
   Glu Val Glu Cys Met Thr Cys Asn Gly Glu Ser Tyr Arg Gly Leu Met
   180 185 190
   Asp His Thr Glu Ser Gly Lys Ile Cys Gln Arg Trp Asp His Gln Thr
   195 200 205
   Pro His Arg His Lys Phe Leu Pro Glu Arg Tyr Pro Asp Lys Gly Phe
   210 215 220
   Asp Asp Asn Tyr Cys Arg Asn Pro Asp Gly Gln Pro Arg Pro Trp Cys
   225 230 235 240
   Tyr Thr Leu Asp Pro His Thr Arg Trp Glu Tyr Cys Ala Ile Lys Thr
   245 250 255
   Cys Ala Asp Asn Thr Met Asn Asp Thr Asp Val Pro Leu Glu Thr Thr
   260 265 270
   Glu Cys Ile Gln Gly Gln Gly Glu Gly Tyr Arg Gly Thr Val Asn Thr
   275 280 285
   Ile Trp Asn Gly Ile Pro Cys Gln Arg Trp Asp Ser Gln Tyr Pro His
   290 295 300
   Glu His Asp Met Thr Pro Glu Asn Phe Lys Cys Lys Asp Leu Arg Glu
   305 310 315 320
   Asn Tyr Cys Arg Asn Pro Asp Gly Ser Glu Ser Pro Trp Cys Phe Thr
   325 330 335
   Thr Asp Pro Asn Ile Arg Val Gly Tyr Cys Ser Gln Ile Pro Asn Cys
   340 345 350
   Asp Met Ser His Gly Gln Asp Cys Tyr Arg Gly Asn Gly Lys Asn Tyr
   355 360 365
   Met Gly Asn Leu Ser Gln Thr Arg Ser Gly Leu Thr Cys Ser Met Trp
   370 375 380
   Asp Lys Asn Met Glu Asp Leu His Arg His Ile Phe Trp Glu Pro Asp
   385 390 395 400
   Ala Ser Lys Leu Asn Glu Asn Tyr Cys Arg Asn Pro Asp Asp Asp Ala
   405 410 415
   His Gly Pro Trp Cys Tyr Thr Gly Asn Pro Leu Ile Pro Trp Asp Tyr
   420 425 430
   Cys Pro Ile Ser Arg Cys Glu Gly Asp Thr Thr Pro Thr Ile Val
   435 440 445
<210> 2
   <211> 442
   <212> PRT
   <213> Human
<220>
   <221> CHAIN
   <222> (1).. (442)
   <223> N-terminal region of alpha-chain in HGF (PyrGlu32-Val478/HGF)
<220>
   <221> MOD_RES
   <222> (130).. (131)
   <223> deletion of 5 amino acids
<400> 2
   Xaa Arg Lys Arg Arg Asn Thr Ile His Glu Phe Lys Lys Ser Ala Lys
   1 5 10 15
   Thr Thr Leu Ile Lys Ile Asp Pro Ala Leu Lys Ile Lys Thr Lys Lys
   20 25 30
   Val Asn Thr Ala Asp Gln Cys Ala Asn Arg Cys Thr Arg Asn Lys Gly
   35 40 45
   Leu Pro Phe Thr Cys Lys Ala Phe Val Phe Asp Lys Ala Arg Lys Gln
   50 55 60
   Cys Leu Trp Phe Pro Phe Asn Ser Met Ser Ser Gly Val Lys Lys Glu
   65 70 75 80
   Phe Gly His Glu Phe Asp Leu Tyr Glu Asn Lys Asp Tyr Ile Arg Asn
   85 90 95
   Cys Ile Ile Gly Lys Gly Arg Ser Tyr Lys Gly Thr Val Ser Ile Thr
   100 105 110
   Lys Ser Gly Ile Lys Cys Gln Pro Trp Ser Ser Met Ile Pro His Glu
   115 120 125
   His Ser Tyr Arg Gly Lys Asp Leu Gln Glu Asn Tyr Cys Arg Asn Pro
   130 135 140
   Arg Gly Glu Glu Gly Gly Pro Trp Cys Phe Thr Ser Asn Pro Glu Val
   145 150 155 160
   Arg Tyr Glu Val Cys Asp Ile Pro Gln Cys Ser Glu Val Glu Cys Met
   165 170 175
   Thr Cys Asn Gly Glu Ser Tyr Arg Gly Leu Met Asp His Thr Glu Ser
   180 185 190
   Gly Lys Ile Cys Gln Arg Trp Asp His Gln Thr Pro His Arg His Lys
   195 200 205
   Phe Leu Pro Glu Arg Tyr Pro Asp Lys Gly Phe Asp Asp Asn Tyr Cys
   210 215 220
   Arg Asn Pro Asp Gly Gln Pro Arg Pro Trp Cys Tyr Thr Leu Asp Pro
   225 230 235 240
   His Thr Arg Trp Glu Tyr Cys Ala Ile Lys Thr Cys Ala Asp Asn Thr
   245 250 255
   Met Asn Asp Thr Asp Val Pro Leu Glu Thr Thr Glu Cys Ile Gln Gly
   260 265 270
   Gln Gly Glu Gly Tyr Arg Gly Thr Val Asn Thr Ile Trp Asn Gly Ile
   275 280 285
   Pro Cys Gln Arg Trp Asp Ser Gln Tyr Pro His Glu His Asp Met Thr
   290 295 300
   Pro Glu Asn Phe Lys Cys Lys Asp Leu Arg Glu Asn Tyr Cys Arg Asn
   305 310 315 320
   Pro Asp Gly Ser Glu Ser Pro Trp Cys Phe Thr Thr Asp Pro Asn Ile
   325 330 335
   Arg Val Gly Tyr Cys Ser Gln Ile Pro Asn Cys Asp Met Ser His Gly
   340 345 350
   Gln Asp Cys Tyr Arg Gly Asn Gly Lys Asn Tyr Met Gly Asn Leu Ser
   355 360 365
   Gln Thr Arg Ser Gly Leu Thr Cys Ser Met Trp Asp Lys Asn Met Glu
   370 375 380
   Asp Leu His Arg His Ile Phe Trp Glu Pro Asp Ala Ser Lys Leu Asn
   385 390 395 400
   Glu Asn Tyr Cys Arg Asn Pro Asp Asp Asp Ala His Gly Pro Trp Cys
   405 410 415
   Tyr Thr Gly Asn Pro Leu Ile Pro Trp Asp Tyr Cys Pro Ile Ser Arg
   420 425 430
   Cys Glu Gly Asp Thr Thr Pro Thr Ile Val
   435 440

## Description

### TECHNICAL FIELD

The present invention relates to neovascularization inhibitors. More particularly, the invention relates to a neovascularization inhibitor (antineovascularization composition) which comprises a protein having a defined regional sequence of the α-chain of hepatocyte growth factor (hereinafter referred to sometimes briefly as HGF) as an active ingredient.

The neovascularization inhibitor of the present invention finds application, based on its inhibitory effect on neovascularization, as a prophylactic or therapeutic agent for various diseases associated with abnormal angiogenesis, such as rheumatoid arthritis, diabetic retinopathy, retinopathy of prematurity, senile macular degeneration and hypercicatrization in wound healing.

### BACKGROUND ART

Neovascularization is a phenomenon such that the vascular endothelial cells, mainly of venules, form a de novo vasculature in response to some stimulus or other. In the normal state of a living body, this phenomenon is indispensable for sustained metabolism of tissues and for functional homeostasis of the body and is generally observed in the process of wound healing, growth of fetal lungs, and evolution of luteinization.

Meanwhile, it is well known that an abnormal angiopoiesis is involved in various diseases inclusive of inflammatory diseases. For example, such diseases as proliferating diabetes, psoriasis vulgaris, rheumatoid arthritis, diabetic retinopathy, senile macular degeneration, overcicatrization in wound healing, etc. and the metastasis and recurrence of carcinomas are reportedly caused by hyperplasia of blood vessels, particularly peripheral capillary vessels [Polverini PJ., Crit. Rev. Oral. Biol. Med., 1995, 6(3), pp.230-247, Review: Forkman J., Nature Med., 1995, 1(1), pp.27-31].

Nakamura et al (FEBS letters, vol. 420, 1997, pages 1-6) describe an antagonist for HGF and designated it HGF/NK4. HGF/NK4 is composed of a polypeptide having the amino acid sequence PyrGlu³²-Val⁴⁷⁸ of the α-chain of HGF and antagonises mitogenic, motogenic and morphogenic and tumor inhibitory activities of HGF by inhibiting the binding of HGF to the receptor and HGF induced tyrosine phosphorylation.

WO 96/40914 relates to two truncated forms of hepatocyte growth factor (HGF). On page 2, line 36 to page 3, line 6 it is stated that one HGF variant is a truncated form of HGF comprising the N-terminal and first two kringle domains of HGF, and specifically antagonises the activity of HGF. The other HGF variant is a truncated form of HGF comprising the N-terminal and the first kringle domain of HGF and is a partial HGF agonist. The HGF variants are called HGF/NK1 and HGF/NK2.

WO 95/29242 relates to angiostatin which is an endothelial inhibitor.

Therefore, as prophylactic or therapeutic drugs for those diseases, a variety of antineovascularization drugs comprising compounds having neovascularization-inhibitory activity as active ingredients have been developed to this day.

The present invention has for its object to provide a novel antineovascularization factor. Another object of the present invention is to provide a neovascularization inhibitor useful for the prevention and treatment of said various diseases arising from hyperplasia of blood vessels.

### BRIEF DESCRIPTION OF THE DRAWINGS

Fig. 1 A is a reversed phase high-performance liquid chromatogram (reversed phase HPLC) (C4) of elastase-treated HGF and B is an eleotrophoretogram (under reducing and non-reducing conditions) of peak fractions on said reversed phase HPLC.
Fig. 2 is a schematic representation of the structures of the α- and β-chains of HGF and the structure of the PyrGlu³² ∼ Val⁴⁷⁸ region of HGF (HGF/NK4) which has been excised by elastase treatment.
Fig. 3 A and B show the dose-dependent rates of binding of ¹²⁵I-HGF and ¹²⁵I-HGF/NK4, respectively, to the rat liver plasma membrane. The insets in Fig.3 A and B show the rates of binding of ¹²⁵I-HGF and ¹²⁵I-HGF/NK4 to said rat liver plasma membrane in Scatchard plots. Fig. 3 C is a diagram showing the rates of binding of unlabeled HGF and unlabeled HGF/NK4 to the rat liver plasma membrane in the presence of ¹²⁵I-HGF (cf. Example 2).
Fig. 4 is a diagram showing the presence or absence of mitogenic activity in HGF and HGF/NK4 (A) and a diagram showing the antagonistic action of HGF/NK4 against the mitogenic activity of HGF. The mitogenic activity information was generated by assaying the DNA synthesis of rat primary-culture hepatocytes. Specifically, Fig. 4 A shows the DNA synthesis of hepatocytes in the presence of HGF or HGF/NK4 and Fig. 4 B shows the effect of HGF/NK4 on the DNA synthesis of hepatocytes in the presence of 60 pM HGF or 1.5 nM epidermal growth factor (EGF) [cf. Example 3].
Fig. 5 is a diagram showing the inhibitory effect of HGF/NK4 on the proliferation of human lung microvascular endothelial cells in the presence or absence (None) of bFGF, HGF or VEGF [Example 7].
Fig. 6 is a diagram showing the inhibitory effect of HGF/NK4 on the proliferation of human skin microvascular endothelial cells in the presence or absence (None) of bFGF, HGF or VEGF [Example 7].
Fig. 7 is a diagram showing the effects of HGF/NK4 (NK4 on the drawing) and anti-HGF antibody (α-HGF Ab on the drawing) on the proliferation of human capillary vessel endothelial cells in the presence of 5% fetal bovine serum (FBS), 5% FBS+bFGF, 5% FBS+VEGF or 5% FBS+HGF [cf. Example 9 A].
Fig. 8 is a diagram showing the effects of HGF/NK4 (NK4 on the drawing) and anti-HGF antibody (α-HGF Ab on the drawing) on the migration of human capillary vessel endothelial cells in the presence of 1% fetal bovine serum (FBS), 1% FBS+bFGF, 1% FBS+VEGF or 1% FBS+HGF [cf. Example 9 B].
Fig. 9 is a set of photographs, in lieu of a drawing, which shows the results of observation of the neovascularization-inhibitory effect of HGF/NK4 on chick embryonic chorioallantoic membrane with a stereoscopic microscope.
Fig. 10 is a photograph, in lieu of a drawing, which shows the inhibitory effect of HGF/NK4 on tumor neovascularization as assayed by an immuno histochemical method.
Fig. 11 indicates that HGF/NK4 has the action to inhibit growth of Lewis lung tumor cells, wherein A is a diagram showing the time course of the volume of a transplanted tumor mass and B is a histogram showing the weight of the transplanted tumor at day 28.
Fig. 12 A is a histogram indicating that HGF/NK4 has the action to inhibit metastasis of Lewis lung tumor cells [Reference Example 1] and Fig. 12 B is a photograph, in lieu of a drawing, which shows the metastasis of the tumor to the lung, indicating clearly that whereas a plurality of lung metastatic foci are present in the saline-treated control group, substantially no lung metastatic foci are detected in the HGF/NK4-treated group.
Fig. 13 indicates that HGF/NK4 has the action to inhibit the growth (A) and metastasis (B) of Jyg mammary tumor cells; A shows the time course of the volume of a transplanted tumor mass and B shows the number of metastatic foci.

### DISCLOSURE OF INVENTION

Searching for a novel neovascularization inhibitor in earnest with the above object in mind, the inventors of the present invention found that a protein containing a defined region of the α-chain of hepatocyte growth factor (HGF) has the action to significantly inhibit neovascularization and have accordingly developed the present invention.

HGF is the very polypeptide which the inventors discovered in 1984 as a novel growth factor for hepatic parenchymal cells (Nakamura, T. et al., Biochem Biophys Res Commun., 1984, 122, pp.1450-1459). Subsequent studies by the present inventors revealed that HGF is a heterodimer consisting of an α-chain having a molecular mass of about 69 kDa and a β-chain having a molecular mass of about 34 kDa and has a unique domain structure comprising an N-terminal hairpin domain and 4 Kringle domains in the α-chain and a serine protease-like domain in the β-chain (Nakamura T. , et al. , Nature 1989, 342, pp.440-443). Though, as of the time of its discovery, HGF was considered to be a growth factor with highly specificity to hepatocytes as the substantive entity of liver regeneration factor, the subsequent studies made since 1989 when recombinant HGFs became available revealed that HGF acts also as a potent mitogen for many kinds of epithelial cells in addition to hepatocytes (Nakamura T., Princess Takamatsu Symp., 1994, 24, pp.195-213. Review). Moreover, the results of further investigations indicated that, in addition to the above cell growth-regulating function, HGF not only has the function of a motogen enhancing cell motility (T. Nakamura, Prog. growth Factor Res., 3, pp.67-85, 1991) but also has novel biological activities inclusive of tumor suppressant activity which inhibits proliferation of many kinds of tumor cells (Higashio K, et al., Biochem Biophys Res Commun., 1990, July 16, 170(1), pp.397-404).

Furthermore, it was elucidated, in 1991, that the functional receptor having a high affinity for HGF is a protooncogene product (c-met product: c-Met) (Bottaro D. P., et al., Science, 1991, Feb., 15, 251(4995), pp.802-804: Naldini L, et al., Oncogene. 1991 Apr, 6(4), pp.501-504), and the present inventors discovered that the N-terminal hairpin domain and first and second Kringle domains of the α-chain are the minimum domains binding to said c-Met/HGF receptor (Matsumoto, K. et al., Biochem. Biophys. Res. Commun., 1991 Dec., 16, 181(2), pp.691-699).

The present inventors did a further study on the heels of the above series of research into HGF and arrived at the novel finding that a polypeptide having said N-terminal hairpin and first through fourth Kringle domains of the α-chain has antagonistic activity against the c-Met/HGF receptor-mediated action of HGF and confirmed that said polypeptide has the action to significantly suppress the neovascularization-inducing action of HGF.

The present invention has been developed on the basis of years of those studies on HGF.

The present invention, therefore, is directed to the following.
1. Use of the polypeptide defined by SEQ ID No. 2 for the production of a pharmaceutical composition containing the polypeptide defined by SEQ ID No. 2 effective for inhibition of neovascularization, wherein said polypeptide has at least one hairpin domain and 4 Kringle domains.
2. A neovascularization inhibitor comprising as an active ingredient the polypeptide defined by SEQ ID No. 2, and having antagonistic activity against the c-Met/HGF receptor-mediated action of HGF, wherein said polypeptide has at least one hairpin domain and 4 Kringle domains.

Representation of amino acids, peptides, nucleotide sequences and others by abbreviations in this specification is principally in conformity with the nomenclature recommended by IUPAC and IUPAC-IUB and the rules set forth in the "Guideline for Preparation of a Specification or the Equivalent Referring to a Nucleotide Sequence or an Amino Acid Sequence" (March 1997, The Examination Standards Office, Coordination Division, the Patent Office of Japan).

Furthermore, the amino acid numbers and positions as mentioned in this specification are based on the amino acid sequence of prepro-HGF (Nakamura T. , et al. , Nature 1989, 342, pp.440-443). In the context of this invention, PyrGlu means pyroglutamate, which is a modified amino acid residue, and PyrGlu³² signifies that, based on the amino acid sequence of prepro-HGF, the 32nd amino acid residue from the N-terminus is pyroglutamate.

The neovascularization inhibitor (antineovascularization composition) of the present invention contains as an active ingredient a polypeptide resulting from the α-chain of HGF and having neovascularization inhibitory activity.

The term "resulting from the α-chain of HGF" is used herein to mean that the entire region or fragments of the α-chain of HGF are contained, whether continuously or discontinuously.

The term neovascularization inhibitory activity means any action that suppresses neovascularization without regard to its mode or mechanism but, in a preferred sense, means the action to suppress the neovasoularization-inducing action of HGF.

As polypeptides having such activity, there can be mentioned polypeptides having an affinity for the c-Met/HGF receptor, which is the receptor of HGF, and the action to competitively antagonize the binding of HGF to said receptor. The preferred are polypeptides which bind to the c-Met/HGF receptor to exhibit antagonistic activity against the c-Met/HGF receptor-mediated action of HGF.

The c-Met/HGF receptor-mediated action of HGF includes c-Met/HGF receptor tyrosine phosphorylating activity, motogenic activity, mitogenic activity and morphogenic activity [Jikken Igaku (Experimental Medicine), Vol. 11, No. 9 (1993)].

Therefore, the polypeptide for use in the present invention is a polypeptide which suppresses or inhibits any of such activities, more particularly a polypeptide having at least one action selected from among the action to suppress/inhibit the HGF-induced c-Met/HGF receptor tyrosine phosphorylation, the action to suppress/inhibit the motogenic activity of HGF, the action to suppress/inhibit the mitogenic activity of HGF and the action to suppress/inhibit the morphogenic activity of HGF. Preferably, a polypeptide having all of the above-mentioned actions can be mentioned.

As reference examples of such polypeptide, there can be mentioned a polypeptide having the amino acid sequence PyrGlu³² ∼ Val⁴⁷⁸ of HGF, more particularly a polypeptide having the amino acid sequence depicted in SEQ ID NO:1. In SEQ ID NO:1, "Xaa" represents "PyrGlu".

This polypeptide can be obtained basically by subjecting HGF to elastase treatment, and consists of the 447 amino acids in the N-terminal region of the α-chain of HGF. Therefore, like the α-chain of HGF, this particular polypeptide has the modified amino acid residue = pyroglutamate at the N-terminus and contains one N-terminal hairpin domain and 4 Kringle domains. In this specification, the above polypeptide is referred to sometimes as HGF/NK4.

As will be shown in the Example which appears hereinafter, this polypeptide exhibits said antagonistic activity by binding to the c-Met/HGF receptor in competition with HGF and has the action to suppress or inhibit the HGF-induced auto-tyrosine-phosphorylation of the c-Met/HGF receptor. Furthermore, this polypeptide has little mitogenic, motogenic or morphogenic activities of its own and has the property to inhibit the mitogenic, motogenic and morphogenic activities of HGF.

However, the polypeptide for use as the active ingredient of the neovascularization inhibitor is a polypeptide resulting from the α-chain of HGF and, inasmuch as it has the action to inhibit the neovascularization-inducing action of HGF, is not limited to said particular polypeptide shown in SEQ ID NO:1.

As specific examples of said polypeptide, there can be mentioned polypeptides having amino acid sequences derived from the amino acid sequence PyrGlu³² ∼ Val¹⁷⁸ of HGF by the deletion, substitution or addition of one, a few or more amino acids and yet having antagonistic activity against the c-Met/HGF receptor-mediated action of HGF.

More preferably, polypeptides equivalent or comparable to said HGF/NK4 in physiological activities (tyrosine phosphorylation inhibiting activity, anti-motogenic activity, anti-mitogenic activity, and anti-morphogenic activity) can be mentioned.

The extent of said "deletion, substitution or addition" of amino acids and the positions involved are not particularly restricted only if the mutant polypeptide still retains the above-mentioned physiological activities. By way of example, a polypeptide resulting from the deletion or addition of one or more than one (or several) amino acids in the N-terminal and/or C-terminal region of said HGF/NK4 and a polypeptide resulting from the deletion or addition of one or more than one (or several) amino acids in the intermediate region of HGF/NK4. Preferably, however, at least one hairpin domain and 4 Kringle domains, which characterize the structure of HGF/NK4, are substantially retained after the mutation.

As a corollary, as a typical mutant peptide, a polypeptide resulting from the substitution, deletion or addition of one or more than one (or several) of the amino acids in the region exclusive of said hairpin domain and 4 Kringle domain can be mentioned. As specific examples of such mutant peptide, there can be mentioned a polypeptide [HGF/NK4 (del 5)] resulting from the deletion of 5 amino acids, namely amino acid Nos. 162-166 (amino acids Nos. 131-135 in SEQ ID NO:1), from the HGF/NK4 polypeptide, that it to say the polypeptide having the amino acid sequence depicted in SEQ ID NO:2.

HGF/NK4 (del 5) for use in the present invention can be respectively produced chemically by a routine method for peptide synthesis or by a routine genetic engineering technique, on the basis of the amino acid sequence information given in SEQ ID NO: 2 or the already-known gene (nucleotide) sequence of HGF.

Preferably, it can be obtained by enzymatic degradation of HGF.

The enzymatic degradation of HGF can be achieved by digesting HGF with an elastase or the like enzyme. Then, this enzymatic digest is purified by the conventional protein purification method, for example high-performance liquid chromatography or SDS-PAGE, and a polypeptide having a given molecular mass is isolated to acquire HGF/NK4. The molecular mass mentioned above may be about 65∼69 kD, preferably about 67 kD, as determined by SDS-PAGE under reducing conditions and about 48-52 kD, preferably about 50 kD, as determined by SDS-PAGE under non-reducing conditions.

HGF for use in said enzymatic degradation is not particularly limited in terms of its source or preparation procedure.

For example, it can be obtained by extraction and purification from liver or other tissues, blood cells such as platelets, leukocytes, etc., plasma or serum of mammals inclusive of man (FEBS, 224, 312, (1987); Proc. Acad. Sci. USA, 86, 5844 (1989)) or by growing primary-culture HGF-producing cells or cell lines and separating and purifying HGF from the resulting culture. As a further alternative, a recombinant HGF can be acquired by a genetic engineering technique which may comprise cloning an HGF-encoding gene in a suitable vector, introducing the vector into suitable host cells (e.g. animal cells) for transfection and harvesting the objective recombinant HGF from a culture supernatant of the resulting cells (e.g. Nature, 342, 440, 1989; JP 1993-111383A; JP 1991-255096A; Biochem., Biophys. Res. Commun., 163, 967, 1989, etc.). The HGF-encoding gene which can be used routinely includes HGF genes derived from mammals inclusive of humans, preferably the HGF gene of the human origin, more preferably human-derived recombinant HGF genes (JP 1993-111383A).

The so-called mutants constructed on the basis of the amino acid sequence of HGF/NK4, such as HGF/NK4 (del 5), can be prepared chemically by a method for peptide synthesis or by a genetic engineering technique starting with an HGF gene.

The technology for mutagenesis includes genetic engineering methods such as site-specific mutagenesis [Methods in Enzymology, 154: 350, pp.367-382 (1987); ditto 100: 468 (1983); Nucleic Acids Res., 12: 9441 (1984); Zoku Seikagaku Jikken Koza 1 "Idenshi Kenkyuho II" (Experimental Biochemistry Series 1 "Methods for Gene Research II") (edited by Japanese Biochemical Society), p105 (1986)], as well as the methods of chemical synthesis, such as the phosphotriester method and phosphoamidate method [J. Am. Chem. Soc., 89: 4801 (1967); do: 91: 3350 (1969); Science, 150: 178 (1968); Tetrahedron Lett., 22: 1859 (1981); do 24: 245 (1983)] and any combination of such techniques.

The neovascularization inhibitor of the present invention is not particularly restricted in dosage form and may be provided in a variety of dosage forms, namely oral preparations such as powders, fine granules, granules, tablets, pills, capsules, solutions, emulsions, suspensions, syrups, etc.; preparations for external application, such as ointments, creams, DDS patches, suppositories, etc.; ophthalmic preparations such as eyedrops, ophthalmic ointments, etc.; injections and drip infusions. These dosage forms can be manufactured by the pharmaceutical procedures well established in the art.

Regarding the manufacture of tablets, the carrier that can be used includes various excipients such as lactose, sucrose, sodium chloride, glucose, urea, starch, calcium carbonate, kaolin, crystalline cellulose, silica, etc.; binders such as simple syrup, glucose solution, starch solution, gelatin solution, carboxymethylcellulose, shellac, methylcellulose, potassium phosphate, polyvinylpyrrolidone, etc.; disintegrators such as dried starch, sodium alginate, agar powder, laminaran powder, sodium hydrogencarbonate, calcium carbonate, polyethoxylated sorbitan fatty acid esters, sodium lauryl sulfate, stearyl monoglyceride, starch, lactose, etc.; disintegration inhibitors such as sucrose, stearic acid, cacao butter, hydrogenated oil, etc.; absorption promoters such as quaternary ammonium bases, sodium lauryl sulfate, etc.; humectants such as glycerin, starch, etc.; adsorbents such as starch, lactose, kaolin, bentonite, colloidal silica, etc.; and lubricants such as purified talc, salts of stearic acid, boric acid powder, polyethylene glycol and so on. Furthermore, where necessary, tablets may be manufactured in the form of coated tablets, i.e. tablets carrying a conventional surface coating, such as sugar-coated tablets, gelatin-coated tablets, film-coated tablets, etc., or in the form of double-layered or multi-layered tablets.

The carrier which can be used in the manufacture of pills include but is not limited to various excipients such as glucose, lactose, starch, cacao butter, hydrogenated vegetable oils, kaolin, talc, etc.; binders such as gum arabic powder, tragacanth powder, gelatin, etc.; and disintegrators such as laminaran and agar, among others.

Capsules can be manufactured by the conventional method which comprises blending said peptide with various carrier substances such as those mentioned above and filling the mixture into hard gelatin capsule shells, soft gelatin capsule shells or the like.

For use in the manufacture of suppositories, the carrier includes polyethylene glycol, cacao butter, higher alcohols, esters of higher alcohols, gelatin and semisynthetic glycerides, among others.

Injections can be manufactured by the conventional technology, for example by dissolving said polypeptide in a suitable solvent, sterilizing the solution for example by filtration, and distributing it into sterile vials. Such injections are preferably isotonic to blood, and the diluent which can be used for provision of such dosage forms includes but is not limited to sterile water, ethyl alcohol, macrogols, propylene glycol, ethoxylated isostearyl alcohol, polyoxylated isostearyl alcohol, polyethoxylated sorbitan fatty acid esters. A sufficient amount of sodium chloride, glucose or glycerin to isotonize such injections may be included in formulations and the conventional solubilizer, buffer, local anesthetic and other additives may also be added.

For the manufacture of an ointment, the ointment base, stabilizer, lubricant, preservative, etc., which are usually employed for such peptides, are formulated and processed in the conventional manner to provide the objective product. The ointment base mentioned above includes liquid paraffin, white petrolatum, bleached beeswax, paraffin and so on. The preservative includes methyl p-hydroxybenzoate, ethyl p-hydroxybenzoate and propyl p-hydroxybenzoate, among others.

Transdermal drug delivery systems can be prepared by spreading said ointment or an equivalent thereof in the form of a paste, cream or gel on the conventional support in the conventional manner. The suitable support includes a woven or nonwoven cloth of cotton, spun rayon or other chemical fiber or a flexible polyvinyl chloride, polyethylene, polyurethane or other sheet or foam sheet.

Furthermore, each of said various pharmaceutical preparations may be supplemented, where necessary, with various pharmaceutically acceptable additives such as a coloring agent, a preservative, a flavoring agent, a corrigent, a sweetener, etc. and/or other medicaments.

In pharmaceutical production runs, a stabilizer is preferably formulated. The stabilizer which can be used includes albumin, globulin, gelatin, mannitol, glucose, dextran and ethylene glycol, to mention but a few examples.

Solutions inclusive of parenteral preparations should be stored frozen or preferably supplied as lyophilized products. Lyophilized preparations are extemporaneously reconstituted with distilled water for injection or the like solvent vehicle.

The amount of the polypeptide to be formulated in the pharmaceutical composition of the present invention is not particularly restricted but can be liberally selected from a broad range. Generally, however, the recommended concentration in the composition is about 0.0002∼0.2 (w/v) %, preferably about 0.001∼0.1 (w/v) %.

The method of administering the pharmaceutical composition is not particularly restricted but can be judiciously selected with reference to the dosage form, patient factors such as age, sex, etc. and severity of illness, among other conditions. By way of illustration, parenteral preparations may be intravenously administered either independently or in admixture with the conventional glucose, amino acid or other infusion. Where necessary, such preparations may be administered alone intramuscularly, intradermally, subcutaneously or intraperitoneally.

The daily dosage of the neovascularization inhibitor of the present invention is dependent on the patient's condition, body weight, age, sex and other factors and cannot be stated in general terms. However, in terms of the amount of the polypeptide of the invention (HGF/NK4 (del 5)), the recommended usual dosage for an adult human is about 0.01-100 mg/day and this dose may be administered in a single dose or in a few divided doses.

The neovascularization inhibitor of the present invention can be applied to the prophylaxis and therapy of a broad spectrum of diseases arising from vascular hyperplasia. Although such diseases are not particularly restricted, there can be mentioned rheumatoid arthritis, psoriasis, Osler-Webber syndrome, myocardial angiopoiesis, telangiectasia, hemophilic joint, angiogenic diseases of the eye (e.g. diabetic retinopathy, retinopathy of prematurity, senile macular degeneration, corneal graft rejection, neovascular glaucoma, retrolental fibroplasia, rubeosis, etc.), angiofibroma, benign tumors (e.g. hemangioma, acoustic neuroma, neurofibroma, trachoma, granuloma pyogenicum, etc.), hematopoietic malignancies such as leukemia, solid cancers, cancer metastasis and wound granulation, among others.

The neovascularization inhibitor of the present invention is further applicable broadly to the prophylaxis and therapy of diseases caused by an excessive or abnormal stimulation of the endothelial cells. Such diseases are not particularly restricted but include, among others, enteric adhesion, Crohn's disease, atherosclerosis, scleroderma, and overcicatrization such as keloid.

Furthermore, the neovascularization inhibitor of the present invention finds application as a drug for conception control based on inhibition of the neovascularization necessary for implantation and finds application as a prophylactic or therapeutic drug for diseases accompanied by angiogenesis, such as cat scratch disease and ulcer, as an pathologic outcome.

### BEST MODE FOR CARRYING OUT THE INVENTION

The following examples are intended to illustrate the present invention in further detail without delineating the technical scope of the invention. Thus, many modifications and changes can be made by those skilled in the art based on the disclosure in this specification without departing from the technical scope of the invention as defined in the claims.

### Example 1 Isolation and purification of HGF/NK4

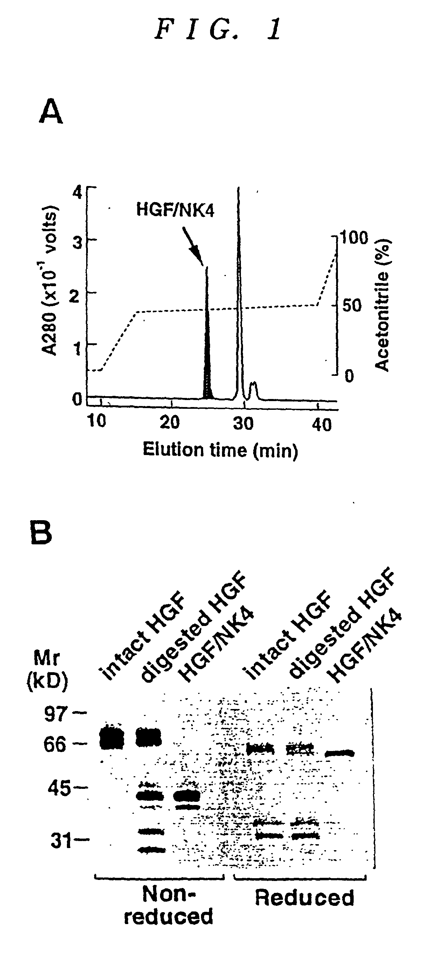

CHO cells transfected with human HGF cDNA were cultured and the recombinant HGF was isolated and purified from the culture (Nature 342, pp.440-443 (1989); Biochem. Biophys. Res. Commun. 172; pp.321-327 (1990)). This recombinant HGF (900 mg) was digested with pancreatic elastase (Sigma) in 0.2 M Tris-HCl buffer (pH 8.8) at 37°C for 20 minutes (enzyme:substrate = 1:100). This digest was purified by reversed-phase high-performance liquid chromatography (HPLC; µ Bondapack C4 column, Waters Japan) on a gradient of 0.05% trifluoroacetic acid-acetonitrile. On the HPLC, three peaks were detected as shown in Fig. 1A.

Then, each of these peak fractions was subjected to SDS-PAGE and protein staining (Fig. 1B). It was found that a first peak is a polypeptide fragment having a molecular mass of 50 kD under nonreducing conditions and of 67 kD under reducing conditions; a second peak corresponds to the undigested heterodimer HGF consisting of an α-chain of 69 kD and a β-chain of 34/32 kD; and a third peak is a fragment having a molecular mass of 33/31 kD under nonreducing conditions and of 34/32 kD under reducing conditions.

In the above description, the expression _/_kD indicates the existence of proteins of the same amino acid sequence but different molecular masses depending on differences in the appended sugar chain.

The foregoing indicated that, upon elastase treatment, HGF is digested into two fragments, namely a fragment (the first peak) comprising a major part of the α-chain but being slightly smaller than the full-length α-chain and a fragment (the third peak) consisting of part of the C-terminal sequence of the α-chain and the full-length β-chain.

For the amino acid sequencing of the purified fragments, the solvent was first evaporated off from each fraction of the eluate and the residue was dissolved in 0.1 M phosphate buffer (pH 7.3) containing 0.05% CHAPS (Sigma) and 1 M NaCl. The amino acid analysis was carried out using Automatic Protein Sequencer 492 (Applied Biosystem Inc.).

To begin with, the analysis for the N-terminal sequence of the first-peak fragment was attempted but failed, suggesting the likelihood that the N-terminus of this fragment was in the pyroglutamate form as it was the case with the N-terminus of HGF. Therefore, this fragment was first treated with pyroglutamate aminopeptidase and then analyzed for the N-terminal sequence. As a result, the sequence, as expressed in single capital letters, was found to be RKRRNTIHEF in agreement with the N-terminal amino acid sequence No. 2 ∼ No. 11 of the α-chain. It was thus found that the N-terminal amino acid of this fragment was glutamine modified in the pyroglutamate form and the structure of the N-terminal region was identical with that of undigested HGF.

Then, for analysis of the C-terminus of this fragment, an amino acid analysis was carried out on the other fragment produced by elastase digestion (the third fragment) for the N-terminus of its partial α-chain. As a result, the amino acid sequence of the N-terminal region of this partial α-chain was found to correspond to the Asn⁴⁷⁹ ∼ Ala⁴⁸⁸ of HGF. This finding indicated that the C-terminus of the first-peak fragment was Val⁴⁷⁸.

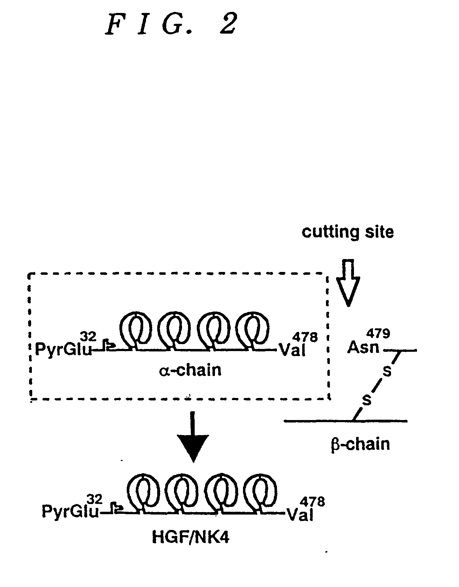

Based on the above results, it was clear that HGF, on elastase treatment, is digested into two fragments, one of which is a fragment consisting in the PyrGlu³² ∼ Val⁴⁷⁸ region having the hairpin domain and 4 Kringle domains of HGF (HGF/NK4) while the other is a fragment consisting of part of the α -chain (16 amino acids starting with Asn⁴⁷⁹) and the β-chain (Fig. 2).

### Example 2 Binding affinity of HGF/NK4 for cell surface receptors

Using the HGF/NK4 prepared in Example 1, its binding affinity for the cell surface receptor was investigated. As a control experiment, the binding affinity of HGF for the cell surface receptor was also investigated. In this connection, the HGF/NK4 and HGF were radio-labeled (¹²⁵I-HGF/NK4 and ¹²⁵I-HGF) by the chloramine-T method, and as the cell surface receptor sample, the plasma membrane prepared from the rat liver was used.

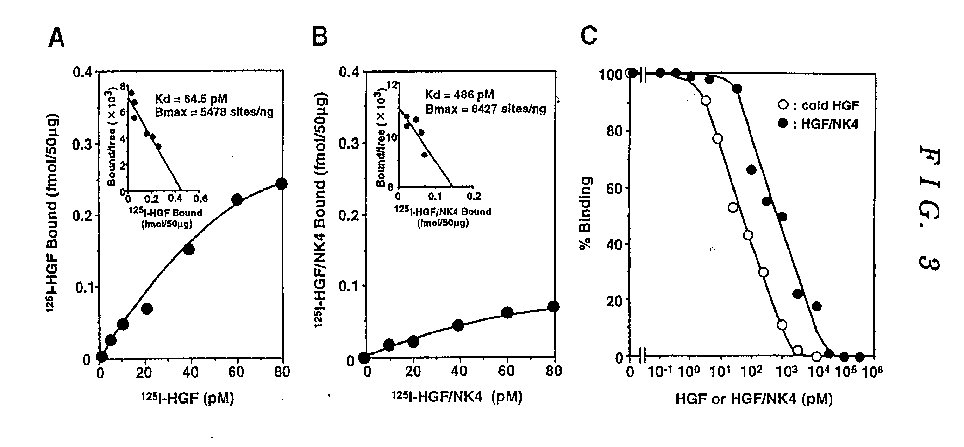

Scatchard analysis revealed that both HGF and HGF/NK4 bind to the cell surface receptors in a concentration-dependent manner up to 80 pM (Fig. 3 A and B), that the Kd and number of HGF receptors were 64.5 pM and 5478 sites/ng, respectively, and that the Kd and number of HGF/NK4 receptors were 486 pM and 6427 sites/ng, respectively.

Then, to investigate whether HGF/NK4 is a competitive antagonist of HGF, the liver plasma membrane (50 µg) was incubated in the presence of ¹²⁵I-HGF alone (60 pM) or a mixture of ¹²⁵I-HGF and a varying amount of unlabeled HGF or unlabeled HGF/NK4. The membrane binding of ¹²⁵I-HGF was completely inhibited by the addition of unlabeled HGF and the 50% inhibitory concentration of unlabeled HGF was 60 pM. Unlabeled HGF/NK4 also inhibited the membrane binding of ¹²⁵I-HGF and the 50% inhibitory concentration of this HGF/NK4 was 600 pM, with the membrane binding of ¹²⁵I-HGF being completely inhibited at 60 nM [Fig. 3 C].

These results suggested that HGF/NK4 has an affinity for the c-Met/HGF receptor, although it is 8-10 times as low as that of HGF and, thus, is an antagonist of HGF.

### Example 3 Mitogenic activity of HGF/NK4 and the inhibitory effect of HGF/NK4 on the mitogenic activity of HGF

The mitogenic activity of HGF/NK4 was evaluated by assaying the DNA synthesis of rat primary-culture hepatocytes. As a control experiment, the mitogenic activity of HGF was similarly evaluated (Nature 342, 440-443 (1989); Biochem. Biophys. Res. Commun. 181, 691-699 (1991)).

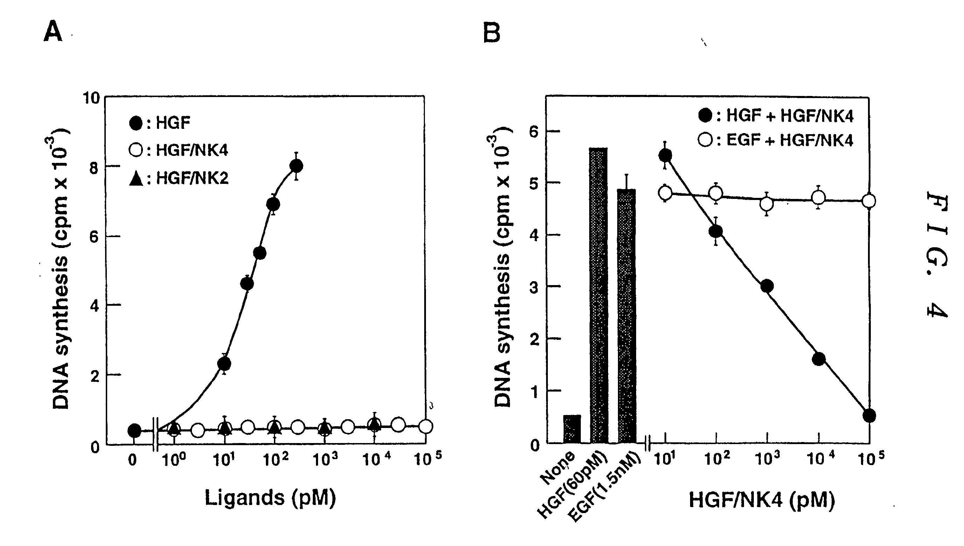

The data are presented in Fig. 4 A. It can be seen from the diagram that whereas HGF promoted the DNA synthesis of hepatocytes dose-dependently, HGF/NK4 did not promote the DNA synthesis even at a high concentration of 100 nM.

On the other hand, when HGF/NK4 was added to an HGF-containing culture medium so that both HGF and HGF/NK4 would be present, HGF/NK4 dose-dependently inhibited the DNA synthesis promoted by HGF, causing a substantially complete inhibition at 60 nM (Fig. 4 B). In contrast, HGF/NK4 showed no inhibitory effect on the DNA synthesis promoted by epidermal growth factor (EGF) (Fig. 4 B).

These results indicated that although HGF/NK4 has no mitogenic activity of its own, it has an action to specifically inhibit the mitogenic action of HGF.

### Example 4 Motogenic activity of HGF/NK4 and the antagonistic action of HGF/NK4 against motogenic activity of HGF

Using MDCK cells, the renal tubule-derived normal epithelial cells, the motogenic activity of HGF/NK4 was evaluated.

The MDCK cells formed a confluent mass of colonies in an HGF-free control medium but addition of HGF (22 pM) to the medium resulted in increased motility of MDCK cells and dispersion of the cells. In contrast, HGF/NK4 did not disperse the cells, with the mutual contact of cells being well maintained. Moreover, when MDCK cells were cultured in the presence of both HGF and HGF/NK4, NGF/NK4 inhibited the HGF-induced cell dispersion.

These results indicated that although HGF/NK4 has no motogenic activity of its own, it has an action to inhibit the motogenic activity of HGF.

### Example 5 Morphogenic activity of HGF/NK4 and the antagonistic action of HGF/NK4 against the morphogenic activity of HGF

To explore whether HGF/NK4 inhibits the morphogenic activity of HGF, MDCK cells, the renal tubule-derived normal epithelial cells, were cultured in collagen gel in the presence of HGF and/or HGF/NK4.

While the MDCK cells formed spherical cell masses in an HGF-free control medium, addition of HGF (55 pM) to the medium induced formation of a branched luminal structure. Addition of HGF/NK4 (55 nM) to this system did not induce such a luminal structure. Moreover, when MDCK cells were grown in the presence of both HGF and HGF/NK4, the cells remained in the form of masses without induction of a luminal structure.

The above results indicated that although HGF/NK4 has no morphogenic activity of its own, it has an action to inhibit the morphogenic activity of HGF.

### Example 6 Inhibitory effect of HGF/NK4 on HGF-induced c-Met tyrosine phosphorylation

The lung tumor cell line A549 is known to have c-Met/HGF receptors expressed and to show enhanced tyrosine phosphorylation of c-Met in response to HGF stimulation. Therefore, it was explored whether HGF/NK4 would inhibit the tyrosine phosphorylation of c-Met by HGF.

Thus, A549 cells were solubilized by stimulation with HGF and/or HGF/NK4 and immunoprecipitated with anti-c-Met antibody. Western blotting was then carried out and the tyrosine phosphorylation of c-Met was studied using anti-phosphotyrosine antibody.

As a result, whereas the stimulation with HGF (110 pM) was found to induce the tyrosine phosphorylation of c-Met, the stimulation with HGF/NK4 (110 nM) induced little phosphorylation. Moreover, HGF/NK4 dose-dependently inhibited the HGF-induced tyrosine phosphorylation of c-Met.

The above results indicated that HGF/NK4 inhibits the HGF-induced tyrosine phosphorylation of c-Met/HGF receptors. It was also suspected that, based on this inhibitory action, HGF/NK4 antagonizes the biological activity of HGF.

### Example 7 Inhibitory effect of HGF/NK4 on growth of vascular endothelial cells

Using human lung microvascular endothelial cells (HMVEC-L; Clonetios) and human dermal microvascular endothelial cells (HMVEC-D; Clonetics) as tester vascular endothelial cells, the growth inhibitory effect of HGF/NK4 on endothelial cells was evaluated.

Thus, using human lung microvascular endothelial cells or human skin microvascular endothelial cells in the logarithmic phase of growth of passage 5-8, a cell suspension was prepared and a gelatin-coated 24-well plate was seeded with 8000 cells per well. After 24 hours, the medium was changed to the fresh one (a 1:1 mixture of EGM (Eagle General Medium) and DMEM (Dulbecco's Modified Eagle Medium) supplied with 5% serum), and four groups of 3 ng/ml bFGF (basic fibroblast growth factor), 10 ng/ml HGF, 10 ng/ml VEGF (vascular endothelial growth factor) and negative control (5% serum-containing solution; None on the drawing) were established. Then, HGF/NK4 in a varying concentration of 0 to 450 nM was added and the plate was incubated under 5% CO₂ at 37°C. After 72 hours, the cells were detached by trypsin coating and counted using a Coulter counter.

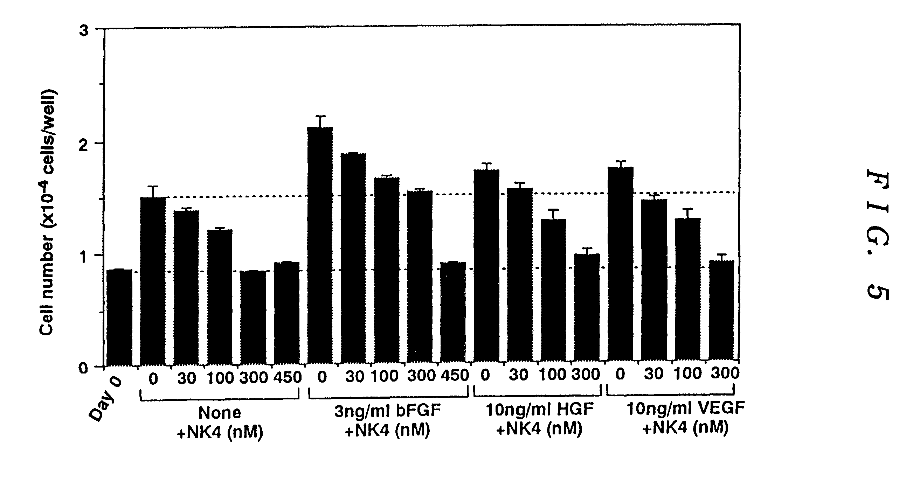

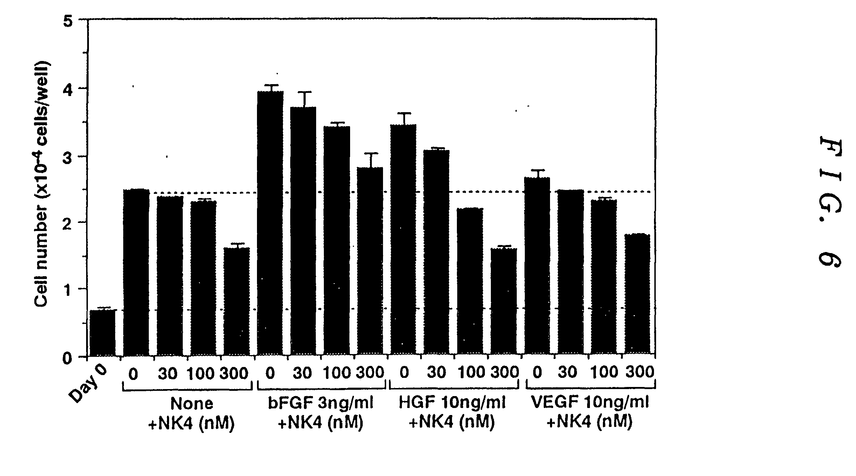

The results for human lung microvascular endothelial cells are shown in Fig. 5 and those for human skin microvascular endothelial cells are shown in Fig. 6.

It can be seen from these diagrams that HGF/NK4 inhibits growth of vascular endothelial cells as induced by stimulation with 3 ng/ml bFGF, 10 ng/ml HGF, 10 ng/ml VEGF and 5% serum, respectively, all concentration-dependently and significantly. These results suggested that HGF/NK4 acts in an inhibitory way not only against HGF-induced growth of vascular endothelial cells but also against the growth induced by other vascular endothelial cell growth factors such as bFGF and VEGF.

### Example 9 Effects of anti-HGF antibody and HGF/NK4 on human capillary vessel endothelial cells

### A. Influence on cell growth

The effects of anti-HGF antibody and HGF/NK4 on growth of human capillary vessel endothelial cells were evaluated.

### Method

Cultured human skin capillary vessel endothelial cells were washed with phosphate-buffered saline (PBS) and detached with trypsin-EDTA (phosphate-buffered saline (PBS) containing 0.05% trypsin and 0.02% EDTA). These endothelial cells were suspended in EBM-2 medium (Clonetics) supplemented with 5% fetal bovine serum (FBS), seeded on a gelatin-coated 24-well plate at a density of 5×10³ cells/cm² and cultured for 24 hours.

The culture was divided into 10 groups according to the following 10 kinds of media based on 5% FBS-EBM-2 medium and the culture medium was changed to the respective media. The symbol α-HGF Ab which appears below stands for anti-HGF rabbit polyclonal antibody.
1. 5% FBS
2. 5% FBS + 3 ng/ml bFGF
3. 5% FBS + 3 ng/ml bFGF + 300 nM HGF/NK4
4. 5% FBS + 3 ng/ml bFGF + 10 µg/ml α-HGF Ab
5. 5% FBS + 10 ng/ml VEGF
6. 5% FBS + 10 ng/ml VEGF + 300 nM HGF/NK4
7. 5% FBS + 10 ng/ml VEGF + 10 µg/ml α-HGF Ab
8. 5% FBS + 3 ng/ml HGF
9. 5% FBS + 3 ng/ml HGF + 300 nM HGF/NK4
10. 5% FBS + 3 ng/ml HGF + 10 µg/ml α-HGF Ab

These media were incubated at 37° C under 5% CO₂ for 72 hours, after which time the cells were detached by trypsin coating and counted with a Coulter counter.

### Results

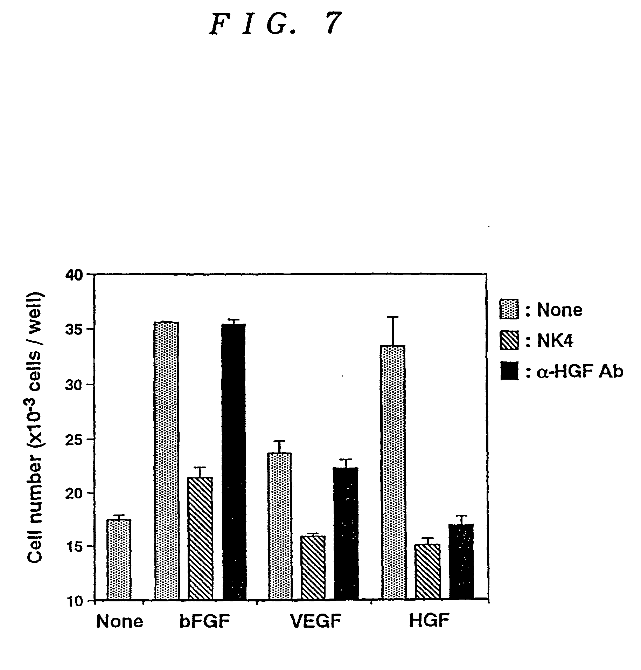

The results are shown in Fig. 7. It can be seen from the diagram that 300 nM HGF/NK4 definitely inhibited growth of vascular endothelial cells as promoted by stimulation with 3 ng/ml bFGF, 10 ng/ml VEGF and 3 ng/ml HGF, respectively. On the other hand, 10 µ g/ml anti-HGF rabbit polyclonal antibody specifically inhibited the HGF-stimulated growth of vascular endothelial cells but did not inhibit the growth of vascular endothelial cells stimulated with bFGF or VEGF. These results suggested that, aside from the HGF-antagonizing action, HGF/NK4 inhibits growth of vascular endothelial cells through some other novel activity.

### B. Influence on cell migration

The effects of anti-HGF antibody and HGF/NK4 on migration of human capillary vessel endothelial cells were evaluated.

### Method

The effect on cell migration was studied by the Boiden chamber method (Yoshida, A. et al., Growth Factors, 1996; 13(1-2): 57-64). Thus, the Boiden chamber test was carried out using a polycarbonate filter with a pore size of 5 µ m which had been coated with 13.4 µg/ml of fibronectin.

First, human capillary vessel endothelial cells were cultured in serum-free EBM-2 medium for 12 hours and suspended in EBM-2 containing 1% fetal bovine serum and the suspension was seeded on said filter at a density of 12×10⁴ cells/cm².

The culture was divided into 10 groups, and bFGF, HGF, VEGF, HGF/NK4 and anti-HGF rabbit polyclonal antibody (α-HGF Ab) were respectively added to the external fluid of the filter cup in these groups as follows.
1. None (1% FBS-EBM-2 medium)
2. 3 ng/ml bFGF
3. 3 ng/ml bFGF + 300 nM HGF/NK4
4. 3 ng/ml bFGF + 10 µg/ml α-HGF Ab
5. 10 ng/ml VEGF
6. 10 ng/ml VEGF + 300 nM HGF/NK4
7. 10 ng/ml VEGF + 10 µg/ml α-HGF Ab
8. 3 ng/ml HGF
9. 3 ng/ml HGF + 300 nM HGF/NK4
10. 3 ng/ml HGF + 10 µg/ml α-HGF Ab

These samples were incubated for 5 hours and the number of cells (per visual field) which had migrated to the underside of the filter was determined under the microscope (x200). For improved accuracy of determination, cell counting was performed in 5 randomly selected visual fields.

### Results

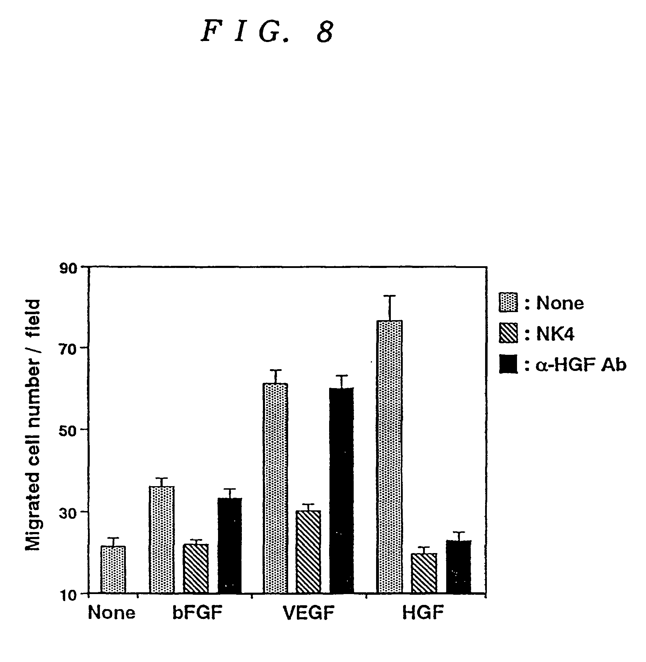

The results are shown in Fig. 8. It is apparent from the diagram that 300 nM HGF/NK4 definitely inhibited the migration of vascular endothelial cells stimulated by 3 ng/ml bFGF, 10 ng/ml VEGF or 3 ng/ml HGF. On the other hand, 10 µg/ml anti-HGF rabbit polyclonal antibody specifically inhibited the HGF-stimulated migration of vascular endothelial cells but did not inhibit the bFGF or VEGF-stimulated migration of these cells. Those results suggested that, in addition to HGF-antagonizing activity, HGF/NK4 inhibits migration of vascular endothelial cells through some other novel activity.

### Example 10 Inhibitory effect of HGF/NK4 on the neovascularization of chick chorioallantoic membrane (CAM)

Fertile chicken eggs were incubated for 4 days, after which the eggshell was drilled in two positions, namely over the air chamber and the lateral side of the shell. From the side hole, 3 ml of the egg white was aspirated and the shell was sealed with a tape. The shell and shell membrane over the air chamber were removed and a silicone ring was set centrally on the chorioallantoic membrane (CAM). Then, an HGF/NK4- or bovine serum albumin (control)-containing methylcellulose disk was set in the silicone ring. After 2 days of incubation at 37° C, the vasculature on the CAM was examined with a stereoscopic microscope. The results are shown in Table 1.

**Table 1**

| | Degree of neovascular invasion |
|---|---|
| HGF/NK4 | + |
| Control | +++ |

The degree of neovascular invasion into the disk was evaluated on the following scale.
-: no invasion
+: about 1∼2 invading vessels
++: about 3-4 invading vessels
+++: 5 or more invading vessels

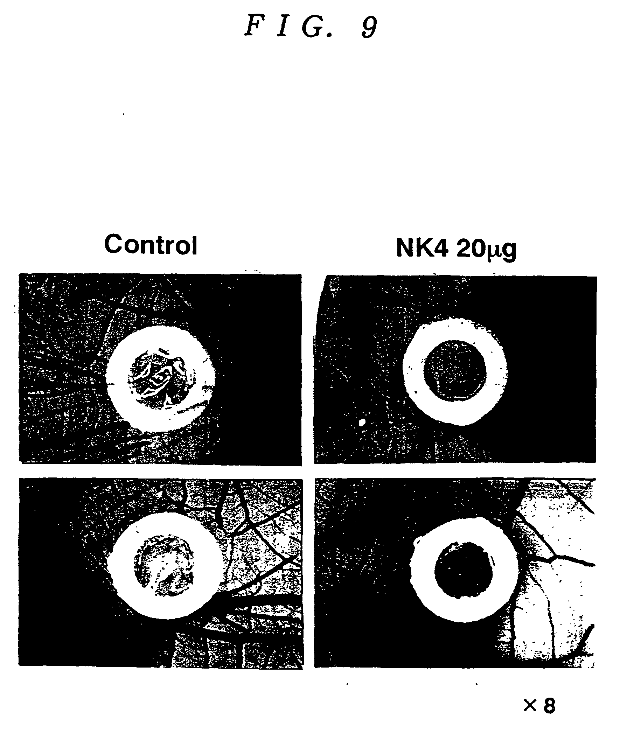

The photographs of the findings obtained in the examination with a stereoscopic microscope, in lieu of a drawing, are presented in Fig. 9.

It will be apparent that whereas the control disk showed a marked neovasoular invasion, the HGF/NK4 disk showed little evidence of neovascular invasion. This finding indicated that HGF/NK4 has neovascularization inhibitory activity.

### Example 11 Inhibitory effect of HGF/NK4 on tumor neovascularization

Using 6∼8-week-old nude mice (BALB/o nu/nu), 5×10⁶ GB-d1 human gallbladder cancer cells were transplanted subcutaneously in the dorsal region. After 7 days, an osmotic pressure pump (Alzet) containing HGF/NK4 or, as control, saline was implanted beneath the dorsal skin and HGF/NK4 or saline was continuously infused into the vicinity of the transplanted cancer cells for 13 days. At week 4 after transplantation, the tumor mass was excised, fixed and sectioned in the routine manner to prepare a tissue specimen. For evaluating neovascularization in the cancer tissue, the tissue section was stained by the immunohistochemical method using anti-von Willebrand factor antibody (Dako). The results are shown in Table 2.

**Table 2**

| | von Willebrand factor stain |
|---|---|
| HGF/NK4 | + |
| Control | +++ |

The degree of neovascularization in the cancer tissue was evaluated on the following scale.
- : no von Willebrand factor-positive microvessels
+ : about 1/4 of microvessels are von Willebrand factor-positive
++: about 1/2 of microvessels are von Willebrand factor-positive
+++: about 3/4 of microvessels are von Willebrand factor-positive
++++: more than 3/4 of microvessels are von Willebrand factor-positive

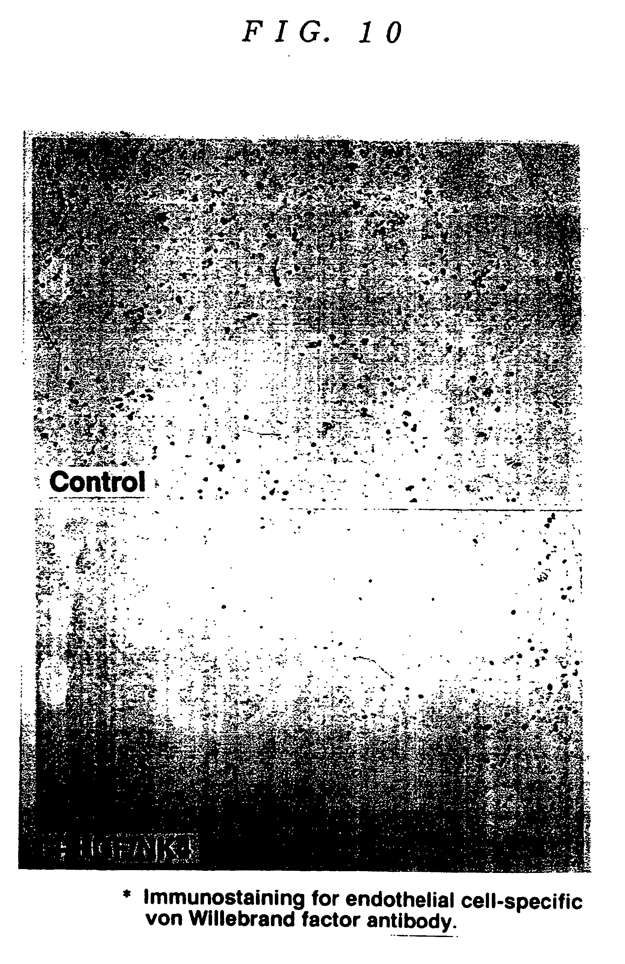

Moreover, the findings in the microscopic examination are presented as photographs, in lieu of a drawing [Fig. 10].

It will be apparent from the above results that a large number of von Willebrand factor-positive microvessels were found in the control saline-infused graft tumor tissue, with no evidence of apoptosis of cancer cells in the tumor mass. In contrast, in the HGF/NK4-perfused graft tumor tissue, tumor neovascularization was remarkably inhibited, with evidence of an extensive apoptosis of cancer cells in the center of the tumor mass. These results indicated that HGF/NK4 inhibits tumor neovascularization in vivo.

### Reference Example 1 Inhibitory effect of HGF/NK4 on tumor growth and metastasis (1)

Using 6∼8-week-old nude mice (BALB/c nu/nu), 5×10⁶ Lewis lung cancer cells were transplanted subcutaneously at the animal back. After 5 days, an osmotic pressure pump (Alzet) containing HGF/NK4 or saline (control) was implanted beneath the dorsal skin and HGF/NK4 or saline was continuously infused into the neighborhood of the tumor graft for 2 weeks. At 28 days after transplantation, the weight and pulmonary metastasis of the transplanted tumor were investigated. The tumor volume was calculated by means of the formula: (minor diameter)² × (major diameter)² × 0.5.

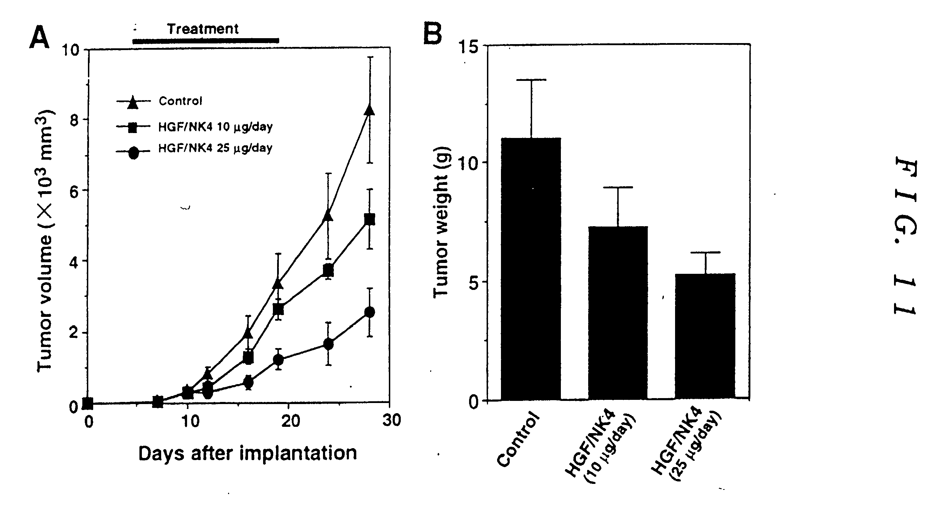

The time course of tumor volume is shown in Fig. 11 A and the weight of the transplanted tumor at day 28 is shown in Fig. 11 B. It will be apparent from these diagrams that whereas the transplanted tumor perfused with saline showed rapid growth at 10 days after transplantation and onwards, HGF/NK4 inhibited tumor growth strongly and dose-dependently.

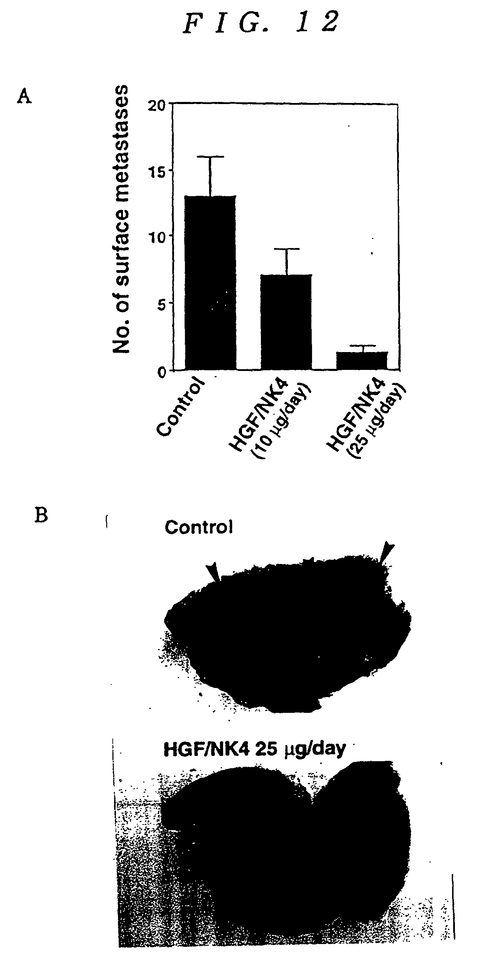

Regarding the pulmonary metastasis of Lewis lung cancer, whereas a large number of pulmonary metastatic focis were observed in the control saline-perfused group, pulmonary metastasis was inhibited remarkably and dose-dependently in the animals perfused with HGF/NK4 (Fig. 12 A, B).

These results indicated that HGF/NK4 has an action to significantly inhibit the growth and metastasis of lung cancer in vivo.

### Reference Example 2 Inhibitory effect of HGF/NK4 on cancer growth and metastasis (2)

Using 6∼8-week-old nude mice (BALB/c nu/nu), 5×10⁶ Jyg mammary cancer cells were transplanted subcutaneously in the dorsal region. After 5 days, an osmotic pressure pump (Alzet) containing HGF/NK4 or saline (control) was implanted beneath the dorsal skin and HGF/NK4 or saline was continuously infused into the neighborhood of the transplanted tumor for 2 weeks. The volume of the tumor graft was serially measured and the number of metastatic foci on the lung surface was counted at 28 days after transplantation.

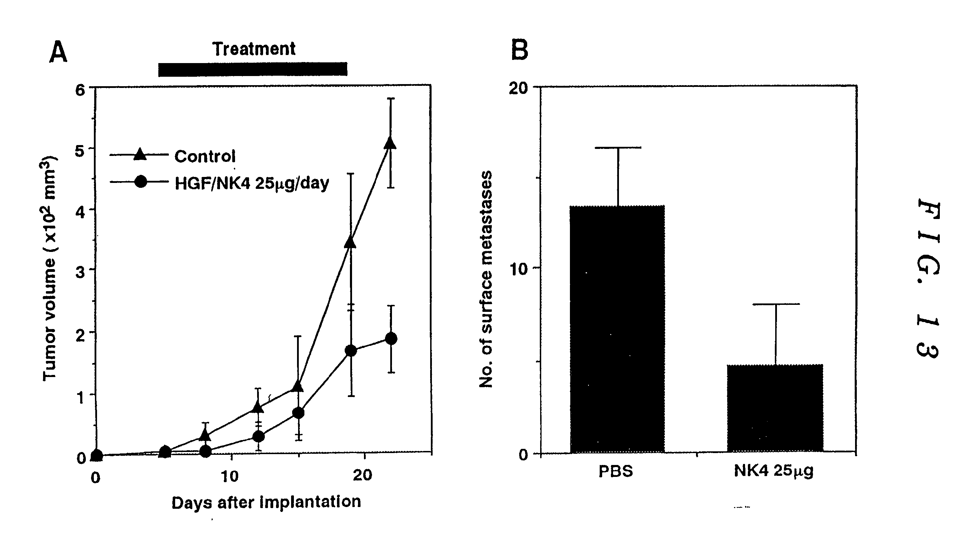

The time course of tumor volume is shown in Fig. 13 A and the number of metastatic foci on the lung surface at day 28 after transplantation is shown in Table 13 B. It will be apparent that whereas the control tumor perfused with saline showed rapid growth at day 10 after transplantation and onwards, HGF/NK4 suppressed growth of the tumor. Regarding pulmonary metastasis, whereas a large number of pulmonary metastatic foci were observed in the control saline-perfused group, pulmonary metastasis was inhibited in the animals perfused with HGF/NK4.

These results indicated that HGF/NK4 has an action to inhibit the growth and metastasis of Jyg mammary cancer significantly in vivo.

### Formulation reference Example 1a

A solution containing the HGF/NK4 prepared in Example 1 (1 mg), mannitol (1 g) and polysolvate 80 (10 mg) in 100 ml of saline was aseptically prepared and distributed into vials, 1 ml per vial. The vial contents were lyophilized and the vials sealed to provide the neovascularization inhibitor of the invention in the form of a lyophilizate.

### Formulation reference Example 2

An aqueous solution containing 1 mg of the HGF/NK4 prepared in Example 1a and 100 mg of human serum albumin was aseptically formulated with 100 ml of 0.02 M phosphate buffer (containing 0.15 M NaCl and 0.01% polysolvate 80; pH 7.4) and the mixture was distributed into vials, 1 ml per vial. The liquid contents of the vials were lyophilized and the vials sealed to provide the neovascularization inhibitor in the form of a lyophilizate.

### Formulation reference Example 3

A solution containing the HGF/NK4 prepared in Example 1a (1 mg), sorbitol (2 g), glycine (2 g) and polysolvate 80 (10 mg) in 100 ml of distilled water for injection was aseptically prepared and distributed into vials, 1 ml per vial. The contents of the vials were lyophilized and the vials sealed to provide the neovascularization inhibitor in the form of a lyophilizate.

### INDUSTRIAL APPLICABILITY

The neovascularization inhibitor of the present invention finds application, based on its neovascularization inhibitory activity, as a prophylactic or therapeutic agent for various diseases associated with abnormal angiopoiesis, such as rheumatoid arthritis, diabetic retinopathy, retinopathy of prematurity, senile macular degeneration, and overcicatrization associated with wound healing.

## Claims

1. Use of the polypeptide defined by SEQ ID No. 2 for the production of a pharmaceutical composition containing the polypeptide defined by SEQ ID No. 2 effective for inhibition of neovascularization, wherein said polypeptide has at least one hairpin domain and 4 Kringle domains.

2. A neovascularization inhibitor comprising as an active ingredient the polypeptide defined by SEQ ID No. 2, and having antagonistic activity against the c-Met/HGF receptor-mediated action of HGF, wherein said polypeptide has at least one hairpin domain and 4 Kringle domains.

## Patentansprüche

1. Verwendung des Polypeptids wie definiert durch SEQ ID Nr. 2 für die Herstellung einer pharmazeutischen Zusammensetzung, enthaltend das Polypeptid definiert durch SEQ ID Nr. 2, effektiv zur Inhibition der Neovaskularisierung, wobei das Polypeptid mindestens eine Hairpindomäne und 4 Kringeldomänen aufweist.

2. Neovaskularisierungsinhibitor, umfassend als aktiven Bestandteil das Polypeptid wie definiert durch SEQ ID Nr. 2 und mit einer antagonistischen Aktivität gegen die c-Met/HGF rezeptorvermittelte Wirkung von HGF, wobei das Polypeptid mindestens eine Hairpindomäne und 4 Kringeldomänen aufweist.

## Revendications

1. Utilisation du polypeptide défini par la SEQ ID No. 2 pour la production d'une composition pharmaceutique contenant le polypeptide défini par la SEQ ID No. 2 efficace pour une inhibition de néovascularisation, dans laquelle ledit polypeptide a au moins un domaine à structure en épingle à cheveux et 4 domaines Kringle.

2. Inhibiteur de néovascularisation comprenant en tant qu'ingrédient actif le polypeptide défini par la SEQ ID No. 2, et ayant une activité antagoniste contre l'action de HGF pour laquelle le récepteur c-Met/HGF sert d'intermédiaire, où ledit polypeptide a au moins un domaine à structure en épingle à cheveux et 4 domaines Kringle.
